# EUROPEAN PATENT SPECIFICATION

(11) **EP 0 519 569 B1**
(45) Date of publication and mention of the grant of the patent: **06.09.1995**
(21) Application number: 92201779.3
(22) Date of filing: 17.06.1992
(51) Int. Cl.: C07C 27/12, C07C 45/33, C07C 29/50, C07C 51/31, C07C 55/14, B01J 29/04

(54) **Catalytic oxidation of hydrocarbons**
Katalytische Oxydation von Kohlenwasserstoffen
Oxydation catalytique d'hydrocarbures

(30) Priority: 20.06.1991 GB 9113343
(43) Date of publication of application: 23.12.1992
(73) Proprietor: SHELL INTERNATIONALE RESEARCH MAATSCHAPPIJ B.V., 2596 HR Den Haag (NL)
(72) Inventor: Kraushaar-Czarnetzki, Bettina, NL-1031 CM Amsterdam (NL); Hoogervorst, Willemina Gerarda Maria, NL-1031 CM Amsterdam (NL)

(56) References cited:
- US-A- 3 529 020
- US-A- 4 567 029

## Description

The invention relates to a chemical process for the catalytic oxidation of hydrocarbons into hydrocarbon oxidation products with molecular oxygen using a cobalt-containing catalyst. More in particular the invention relates to such a process in which the cobalt containing catalyst comprises a cobalt-containing molecular sieve material.

Processes for the catalytic oxidation of hydrocarbons with molecular oxygen using a cobalt-containing catalyst are known in the art.

DE-A-3 733 782 (Zakladi Azotowe Im.) discloses a process for oxidising cyclohexane to cyclohexanol and cyclohexanone with molecular oxygen under pressure at a temperature between 150 to 200 °C using cobalt naphthenate as a homogeneous catalyst.

JP-A-89/294 646 (Mitsubishi Kasei Corp.) discloses a process for the oxidation of cyclohexane to cyclohexanol and cyclohexanone with molecular oxygen in the presence of catalyst comprising a magnesium oxide or double oxide such as Mg-Al-hydrotalcite, MgO-Al₂O₃ or a zeolite ion exchanged with Mg²⁺, and being charged with Co ions. Reaction temperature and pressure are from 110 to 180 °C and below 100 kg/cm² respectively. In a similar oxidation reaction JP-A-88/303 936 discloses the use of a certain phyllosilicate catalysts in which cations have been exchanged with cobalt ions.

These processes suffer from the disadvantage that the cobalt ions at least after the oxidation reaction are substantially present in reaction liquid and must be recovered therefrom. Cobalt ions introduced into a molecular sieve by cation exchange are present on the surface of the ion exchange material and only loosely attached thereto mainly by electrostatic forces. Such cobalt ions are leached out easily during the oxidation reaction contaminating the reaction product and the catalytic activity of the molecular sieve is decreased. In fact the actual catalysis may then well be due to the cobalt ions in solution.

Also reaction temperatures in the range of 150-200 °C are considered relatively high because they may tend to produce reaction products of darker colour and to induce chain length degradation in the reaction products.

Especially cheap hydrocarbon oxidation products in large quantities are a valuable product for further chemical processing to polyamides and polyesters and further as a fuel improver. Therefore there is a need for an economically attractive industrial bulk manufacturing process, using cheap hydrocarbon starting materials and operating under attractive economical, environmental and safe conditions, i.e. using rather simple equipment and resulting in a significant reduction of the cost price of these products. Therefore considerable research and development efforts have been made for a further improved oxidation process.

According to the present invention hydrocarbons are oxidized to hydrocarbon oxidation products with a source of molecular oxygen (especially air as a source of molecular oxygen) using a cobalt-containing catalyst which is a molecular sieve material having the cobalt ions substantially incorporated in the crystal lattice of the molecular sieve. It will be clear that by using such a catalytic material that the active cobalt ions are well integrated in the crystal lattice and more or less permanently fixed therein. Consequently these ions substantially retain their position in the crystal lattice also during the oxidation reaction so that catalytic activity is not appreciably decreased and the reaction product is substantially uncontaminated by cobalt ions.

In a preferred embodiment of the invention the oxidation reaction is started with a molecular sieve material in which the cobalt ions are substantially trivalent. It is, however, quite possible to start the process according to the present invention using molecular sieve material containing mainly divalent cobalt ions, since these ions can readily be oxidized to trivalent cobalt ions in situ. When using molecular sieve material containing divalent cobalt ions, it may be used in its acid exchanged form.

It is recommended to use cobalt containing aluminophosphate based molecular sieve material of the structure type 5, 31, 36, 37, 40 and/or 50 as defined in "New developments in Zeolite Science and Technology", (Eds. Y.Murakamami, A.Lijima and J.W.Ward), Stud. Surf. Sci. Catal., Vol. 28, Elsevier, Tokyo, 1986 pp. 103-111 and J.M. Bennett and B.K. Marcuse in "Innovation in zeolite material science", (Ed P.J.Grobet et al), Series: Studies on surface science and catalysis, Vol. 37, Elsevier, Amsterdam (1988) p. 269 ff. It is particularly recommended to use in the oxidation reaction a molecular sieve of the class comprising:
Co-containing MeAPO's (as described as CoAPO's in US-A-4 567 029)
Co-containing MeAPSO's (as described as CoAPSO's in EP-A-161 489)
Co-containing XAPO's (as described as FeTiCoAPO's in US-A-4 952 384
Co-containing SENAPO's (as described in EP-A-158 350).

Very useful in the oxidation reaction according to the present invention are CoAPO-5 molecular sieve materials and similar cobalt aluminophosphate species. CoAPO-5 and similar molecular sieves and processes for their preparation are described in US-A-4 567 029 (Union Carbide Corporation), in particular in examples 89-92. Preferably the molecular sieve employed in the oxidation reaction is free from template and e.g. in the calcined form. To minimize transport problems it is also recommended to use a molecular sieve material having a pore diameter sufficiently large for adsorption of the hydrocarbons being oxidized. Therefore in the case of cyclohexane a pore diameter of at least 0.65 nm is recommended, whereas for the oxidation of straight chain hydrocarbons a molecular sieve with a pore diameter of at least 0.38 nm is recommended.

More in general the present invention comprises the use of molecular sieve material containing from 0.1 to 10% by weight of cobalt in the lattice. Furthermore it is preferred to use, especially in batchwise oxidation processes, an amount of cobalt-containing molecular sieve material which ranges between 0.5 and 25, preferably between 1 and 10 percent by weight calculated on the amount of hydrocarbon to be oxidized. In the case of continuous processing the amount of molecular sieve material used can be considerably lower.

It is preferred to carry out the oxidation at a temperature of from ambient to 200 °C, more preferably between 80 and 140 °C and at a reaction pressure up to 5000 kPa, more preferably between 100 and 2000 kPa. Air is the most convenient source of molecular oxygen for use in the present process. However, lower oxygen partial pressures by dilution, or higher oxygen partial pressures by concentration, or pure oxygen may also be used. The oxygen partial pressure may be used to direct the oxidation to specific reaction products.

In accordance with the present invention diverse hydrocarbons can be oxidized, more in particular the process is useful to oxidize hydrocarbons of the class comprising alkanes and cycloalkanes, which may be substituted with groups which do not interfere with the process. Preferred alkanes have from 1 to 10 carbon atoms, more preferably from 3 to 6 carbon atoms. Preferred cycloalkanes have from 3 to 8 carbon atoms.

Oxidized products comprise alcohols, aldehydes, ketones and carboxylic acids. The oxidation is non-degrading i.e. the original carbon chain length is mostly retained. E.g. when the preferred starting material cyclohexane is oxidized the reaction product consists predominantly of cyclohexanol, cyclohexanone and adipic acid, where cyclohexylacetate as cyclohexanol esterification product may e.g. be present in case acetic acid is present during the oxidation.

The present invention also permits the preparation of naphtha oxygenates useful as a blending additive for reformulated gasoline having boosted octane number.

It is often preferred for more convenient processing to employ an acid as a promoter during the oxidation reaction. Inorganic acids e.g. phosphoric acid, and carboxylic acids, optionally chlorinated or fluorinated can be used. Lower (C1-C6) carboxylic acids, in particular acetic acid are preferred. Preferred acids will be miscible with the hydrocarbon substrate and resistant to oxidation under the conditions of the present process. Boric acid or bulky carboxylic acids forming stable esters may be used, should it be desired that the oxidation reaction will not substantially proceed beyond the formation of the alcohol in the first step.

The process according to the present invention can be carried out discontinuously or batchwise, semi-continuously (e.g. cascade method) or continuously, preferably by passing the hydrocarbon to be oxidized in the presence of molecular oxygen over a fluidized bed of cobalt-containing molecular sieve material, in a suitable reactor. This process permits prolonged use of the catalyst bed, which functions as a true heterogenous catalyst because the cobalt ions remain entrapped in the crystal lattice of the molecular sieve during the oxidation and thereafter. This process also results in a less contaminated reaction product than hitherto known for cobalt catalysed oxidation reactions. Regeneration of the catalyst can be achieved easily by washing or calcination.

### Example 1

A mixture comprising 40 ml (31.13 g) cyclohexane and 10 ml (10.5 g) of acetic acid was transferred to Teflon-lined autoclave of 500 ml volume. Then, 5 g of CoAPO-5, containing 1.9% (w.w.) Co and having an anhydrous composition which could be expressed in molar oxide ratios as [0.08 CoO . 0.99 Al₂O₃ . 1 P₂O₅] and which previously had been calcined for 2 hrs at 550 °C were added. The autoclave was charged with air (containing 21 vol% of oxygen) to a pressure of 6.5 x 10⁵ Pa and the reaction was performed under continuous stirring of the slurry at a temperature of 373 K. After 3 hours the autoclave was cooled, and the solid catalyst was separated from the liquid by filtration. The liquid was found to contain 4.1 g of the acetic acid ester of cyclohexanol, 2 g of cyclohexanone, 0.1 g of adipic acid and 0.9 g of other oxygenates derived from cyclohexane, among which some degradation products (e.g., n-pentylacetate and n-butylacetate). The gaseous phase was found to contain 0.6 vol% of residual oxygen.

After the reaction investigation of the catalyst showed that both crystallinity and composition were not effected by the oxidation of cyclohexane and the catalyst had retained its activity. Consequently no cobalt had leached into the liquid phase.

### Comparative Experiment

The procedure described above in Example 1 was repeated using as a catalyst template-free SAPO-5 having an anhydrous composition expressed in molar oxide ratios of [0.21 SiO₂ . 1Al₂O₃ . 0.88 P₂O₅] and loaded with 2.6% (w.w.) of cobalt by pore volume impregnation with an aqueous cobalt acetate solution, followed by drying. The product mixture was found to contain 0.9 g of acetic acid ester of cyclohexanol, 0.75 g of cyclohexanone, 0.1 g of adipic acid and 1.1 g of other oxygenates derived from cyclohexane, including degradation products.

After the reaction the catalyst contained only 0.2% (w.w.) of cobalt due to leaching and dissolution in the liquid reaction product.

## Claims

1. Process for the catalytic oxidation of hydrocarbons into hydrocarbon oxidation products with a source of molecular oxygen using a cobalt-containing catalyst in which the cobalt-containing catalyst is a molecular sieve material having the cobalt ions substantially incorporated in the crystal lattice of the molecular sieve.

2. Process according to claim 1, in which the molecular sieve contains from 0.1 to 10 percent by weight of cobalt.

3. Process according to claim 1 or 2 in which the catalyst is a cobalt-containing aluminophosphate based molecular sieve material of the structure type 5, 31, 36, 37, 40 and/or 50.

4. Process according to any of the preceding claims in which the oxidation is carried at a temperature of from ambient to 200 °C and a pressure between 100 and 2000 kPa.

5. Process according to any of the preceding claims in which the hydrocarbon being oxidized is comprised by the class of alkanes having from 1 to 10 carbon atoms and cycloalkanes having from 3 to 8 carbon atoms.

6. Process according to any of the preceding claims in which the hydrocarbon being oxidized is cyclohexane.

7. Process according to any of the preceding claims in which the oxidation is carried out in the presence of an acid as a promoter.

8. Process according to any of the preceding claims in which the oxidation is carried by passing the hydrocarbon in the presence of molecular oxygen over a fluidized bed of cobalt-containing molecular sieve material.

## Patentansprüche

1. Verfahren zur katalytischen Oxidation von Kohlenwasserstoffen zu Kohlenwasserstoffoxidationsprodukten mit einer Quelle von molekularem Sauerstoff unter Verwendung eines cobalthaltigen Katalysators, wobei es sich bei dem cobalthaltigen Katalysator um ein Molekularsiebmaterial handelt, bei dem die Cobaltionen weitgehend im Kristallgitter des Molekularsiebs eingebaut sind.

2. Verfahren nach Anspruch 1, bei dem das Molekularsieb 0,1 bis 10 Gewichtsprozent Cobalt enthält.

3. Verfahren nach Anspruch 1 oder 2, wobei es sich bei dem Katalysator um ein cobalthaltiges, auf Aluminophosphat basierendes Molekularsiebmaterial mit einer Struktur vom Typ 5, 31, 36, 37, 40 und/oder 50 handelt.

4. Verfahren nach einem der vorhergehenden Ansprüche, wobei man die Oxidation bei einer Temperatur von Umgebungstemperatur bis 200°C und einem Druck zwischen 100 und 2000 kPa durchführt.

5. Verfahren nach einem der vorhergehenden Ansprüche, bei dem der zu oxidierende Kohlenwasserstoff von der Klasse der Alkane mit 1 bis 10 Kohlenstoffatomen und der Cycloalkane mit 3 bis 8 Kohlenstoffatomen umfaßt wird.

6. Verfahren nach einem der vorhergehenden Ansprüche, wobei es sich bei dem zu oxidierenden Kohlenwasserstoff um Cyclohexan handelt.

7. Verfahren nach einem der vorhergehenden Ansprüche, wobei man die Oxidation in Gegenwart einer Säure als Promoter durchführt.

8. Verfahren nach einem der vorhergehenden Ansprüche, wobei man die Oxidation durch Hinüberleiten des Kohlenwasserstoffs in Gegenwart von molekularem Sauerstoff über ein Wirbelbett aus cobalthaltigem Molekularsiebmaterial durchführt.

## Revendications

1. Procédé pour l'oxydation catalytique d'hydrocarbures en produit d'oxydation d'hydrocarbures au moyen d'une source d'oxygène moléculaire, en mettant en oeuvre un catalyseur renfermant du cobalt, procédé dans lequel un catalyseur renfermant du cobalt est un matériau de type tamis moléculaire ayant des ions cobalt essentiellement incorporés dans le réseau cristallin du tamis moléculaire.

2. Procédé selon la revendication 1, dans lequel le tamis moléculaire renferme de 0,1 à 10% en poids de cobalt.

3. Procédé selon la revendication 1 ou 2, dans lequel le catalyseur est un matériau de type tamis moléculaire à base d'aluminophosphate renfermant du cobalt du type de structure 5, 31, 36, 37, 40 et/ou 50.

4. Procédé selon l'une quelconque des revendications précédentes, dans lequel l'oxydation est mise en oeuvre à une température entre la température ambiante et 200°C et sous une pression entre 100 et 2000 kPa.

5. Procédé selon l'une quelconque des revendications précédentes, dans lequel l'hydrocarbure devant être oxydé est compris dans la classe des alcanes ayant de 1 à 10 atomes de carbone et des cycloalcanes ayant de 3 à 8 atomes de carbone.

6. Procédé selon l'une quelconque des revendications précédentes dans lequel l'hydrocarbure devant être oxydé est le cyclohexane.

7. Procédé selon l'une quelconque des revendications précédentes, dans lequel l'oxydation est mise en oeuvre en présence d'un acide en tant que promoteur.

8. Procédé selon l'une quelconque des revendications précédentes, dans lequel l'oxydation est mise en oeuvre en faisant passer l'hydrocarbure, en présence d'oxygène moléculaire sur un lit fluidisé en matériau de type tamis moléculaire renfermant du cobalt.
